(19) Europäisches Patentamt
European Patent Office
Office européen des brevets

(11) Veröffentlichungsnummer: **0 115 640 A2**

(12) # EUROPÄISCHE PATENTANMELDUNG

(21) Anmeldenummer: **83113222.0**

(22) Anmeldetag: **30.12.83**

(51) Int. Cl.³: **C 07 D 403/04**, C 07 D 403/12, A 61 K 31/415

(30) Priorität: **12.01.83 DE 3300795**

(43) Veröffentlichungstag der Anmeldung: **15.08.84 Patentblatt 84/33**

(84) Benannte Vertragsstaaten: **AT BE CH DE FR GB IT LI LU NL SE**

(71) Anmelder: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Perzborn, Elisabeth, Dr., Am Tescherbusch 13, D-5600 Wuppertal 11 (DE)**
Erfinder: **Seuter, Friedel, Dr., Moospfad 16, D-5600 Wuppertal 1 (DE)**

(54) **Substituierte 4-Imidazolyl-pyrazole, Verfahren zu ihrer Herstellung sowie diese enthaltende Arzneimittel.**

(57) Neue substituierte 4-Imidazolyl-pyrazole, mehrere Verfahren zu ihrer Herstellung sowie ihre Verwendung als Arzneimittel, insbesondere in Mitteln mit antithromboembolischer Wirkung.

EP 0 115 640 A2

BAYER AKTIENGESELLSCHAFT 5090 Leverkusen, Bayerwerk

Zentralbereich
Patente, Marken und Lizenzen Sft/Kü-c
Ia

Substituierte 4-Imidazolyl-pyrazole, Verfahren zu ihrer Herstellung sowie diese enthaltende Arzneimittel

Gegenstand der vorliegenden Erfindung sind neue substituierte 4-Imidazolyl-pyrazole der allgemeinen Formel (I),

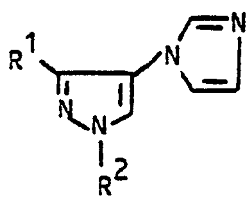

(I)

in welcher

$R^1$ für einen geradkettigen oder verzweigten Alkylrest mit 1 bis 12 Kohlenstoffatomen; für einen jeweils gegebenenfalls einfach oder mehrfach, gleich oder verschieden substituierten Cycloalkylrest mit 3 bis 7 Kohlenstoffatomen oder Cycloalkylalkylrest mit 3 bis 7 Kohlenstoffatomen im Cycloalkylteil und 1 bis 4 Kohlenstoffatomen im Alkylteil, wobei als Substituenten genannt seien: Halogen, Alkyl und Alkoxy mit jeweils 1 bis 4 Kohlenstoffatomen; für einen gegebenenfalls einfach oder mehrfach, gleich oder verschieden substituierten Phenoxyalkylrest mit 1 bis 4 Kohlenstoffatomen im Alkylteil, wobei als Phenylsubstituenten die bei Y genannten Phenylsubstituenten infrage kommen; oder für eine der Gruppierungen

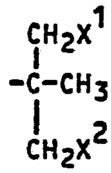 , 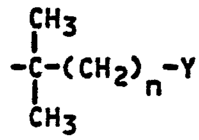 oder 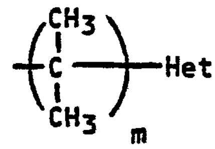 steht,

wobei

$X^1$ und $X^2$ Halogenatome bedeuten,

Y für Halogen; Hydroxy; für einen Alkoxy- oder Alkylthiorest mit jeweils 1 bis 4 Kohlenstoffatomen; für einen Halogenalkoxy- oder Halogenalkylthiorest mit jeweils 1 bis 2 Kohlenstoffatomen und 1 bis 5 gleichen oder verschiedenen Halogenatomen, wie Fluor- und Chloratomen; für einen Alkenyl- oder Alkinylrest mit jeweils 2 bis 6 Kohlenstoffatomen; für einen Alkoxycarbonyl- oder Alkylcarbonyloxyrest mit jeweils 1 bis 4 Kohlenstoffatomen im Alkylteil; für einen gegebenenfalls einfach oder zweifach, gleich oder verschieden durch Alkyl mit 1 bis 4 Kohlenstoffatomen oder jeweils gegebenenfalls einfach bis dreifach, gleich oder verschieden substituiertes Phenyl oder Phenylalkyl mit 1 bis 2 Kohlenstoffatomen im Alkylteil, wobei jeweils der Phenylrest als Substituenten Halogen, Alkyl mit 1 bis 4 Kohlenstoffatomen oder Trifluormethyl enthalten kann, substituierten Carbamoyloxyrest, für einen Aldoxim- oder Ketoxim-Rest oder deren Ether-Derivate; für einen jeweils gegebenenfalls einfach oder mehrfach, gleich oder verschieden substituierten Phenyl-, Phenoxy-, Phenylthio-, Phenylalkoxy- oder Phenylalkylthiorest mit jeweils 1 bis 4 Kohlenstoffatomen im Alkylteil, wobei jeweils der Phenylrest als Substituenten Halogen, Alkylreste mit 1 bis 4 Kohlenstoffatomen, Hydroxy, Alkoxy oder Alkylthioreste

mit jeweils 1 bis 2 Kohlenstoffatomen, Halogenalkyl-, Halogenalkoxy- oder Halogenalkylthioreste mit jeweils 1 bis 2 Kohlenstoffatomen und 1 bis 5 gleichen oder verschiedenen Halogenatomen, wie Fluor- und Chloratomen, Dialkylaminoreste mit 1 bis 4 Kohlenstoffatomen in jedem Alkylteil, Hydroxycarbonyl-, Alkoxycarbonyl-, Alkylcarbonyl- oder Alkylcarbonyloxyreste mit jeweils 1 bis 4 Kohlenstoffatomen in jedem Alkylteil, Nitro oder Cyano enthalten kann; oder für einen gegebenenfalls einfach oder mehrfach, gleich oder verschieden durch Alkylreste mit 1 bis 2 Kohlenstoffatomen und/oder Halogen substituierten 5- oder 6-gliedrigen Heteroarylrest mit 1 bis 2 Heteroatomen, wie vorzugsweise Stickstoff-, Sauerstoff- und Schwefelatom, steht;

Het einen, gegebenenfalls durch Alkylreste mit 1 bis 4 Kohlenstoffatomen oder durch jeweils gegebenenfalls einfach oder mehrfach, gleich oder verschieden substituierte Phenyl- oder Phenoxyalkylreste mit 1 bis 4 Kohlenstoffatomen im Alkylteil einfach oder mehrfach, gleich oder verschieden substituierten, 5- oder 6-gliedrigen heteroaliphatischen Rest mit Sauerstoff und/oder Schwefel als Heteroatome darstellt, wobei die Phenylreste als Substituenten Halogen, Alkylreste mit 1 bis 4 Kohlenstoffatomen, Alkoxy- oder Alkylthioreste mit jeweils 1 bis 2 Kohlenstoffatomen und 1 bis 5 gleichen oder verschiedenen Halogenatomen, wie vorzugsweise Fluor- und Chloraatomen enthalten können;

n eine der Zahlen 0, 1 oder 2 bedeutet und

m für 0 oder 1 steht; ferner

$R^2$ für Wasserstoff; für einen geradkettigen oder verzweigten Alkylrest mit 1 bis 12 Kohlenstoffatomen; für einen Alkenyl- oder Alkinylrest mit jeweils 3 bis 6 Kohlenstoffatomen; für einen Alkylcarbonyl- oder Alkoxycarbonylrest mit jeweils 1 bis 4 Kohlenstoffatomen im Alkylteil; für einen jeweils gegebenenfalls einfach oder mehrfach, gleich oder verschieden substituierten Phenyl- oder Benzylrest, wobei als Substituenten die bei Y bereits genannten Phenylsubstituenten infrage kommen; oder für einen gegebenenfalls einfach oder mehrfach, gleich oder verschieden durch Alkylreste mit 1 bis 2 Kohlenstoffatomen und/oder Halogen substituierten 5- oder 6-gliedrigen Heteroarylrest mit 1 bis 2 Heteroatomen, wie Stickstoff-, Sauerstoff- oder Schwefelatomen steht,

sowie pharmakologisch unbedenkliche Säureadditionssalze von Verbindungen der Formel (I).

Erfindungsgemäß bevorzugt sind diejenigen Verbindungen der Formel (I), in denen

$R^1$ für einen geradkettigen oder verzweigten Alkylrest mit 3 bis 8 Kohlenstoffatomen; für einen jeweils gegebenenfalls einfach bis dreifach, gleich oder verschieden substituierten Cycloalkylrest mit 3 bis 7 Kohlenstoffatomen oder Cycloalkylmethylrest mit 3 bis 7 Kohlenstoffatomen im Cycloalkylteil, wobei als Substituenten Chlor, Brom, Methyl, Ethyl, Isopropyl, Methoxy und Ethoxy in Frage kommen; für einen jeweils einfach oder mehrfach, gleich oder verschieden substituierten Phenoxymethyl- oder Phenoxyethylrest, wobei als Phenylsubstituenten die bei Y genannten Phenylsubstituenten infrage kommen; oder für eine der Gruppierungen

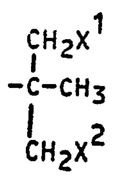 , 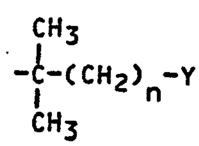 oder 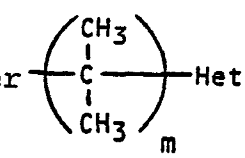

steht, wobei

$X^1$ und $X^2$ ein Fluor-, Chlor- oder Bromatom bedeuten;

Y für Fluor, Chlor, Brom, Hydroxy, einen geradkettigen oder verzweigten Alkoxy- oder Alkylthiorest mit jeweils 1 bis 4 Kohlenstoffatomen,

einen Trifluormethoxy-, Trifluormethylthio-, Vinyl-, Allyl-, Acetylenyl-, Propargyl-, Alkoxycarbonyl oder Alkylcarbonyloxyrest mit jeweils 1 bis 4 Kohlenstoffatomen im Alkylteil, einen Alkyl- oder Dialkylcarbamoyloxyrest mit jeweils 1 bis 2 Kohlenstoffatomen in jedem Alkylteil, einen Phenyl- oder Phenyl-alkyl-carbamoyloxyrest mit 1 bis 2 Kohlenstoffatomen im Alkylteil, wobei der Phenylrest jeweils einfach bis dreifach, gleich oder verschieden durch Fluor, Chlor, Methyl oder Trifluormethyl substituiert sein kann, für einen Hydroximinomethyl- oder Alkoximinomethylrest mit 1 bis 4 Kohlenstoffatomen im Alkoxyteil, für einen 1-Hydroximinoethyl- oder 1-Alkoxyiminoethylrest mit 1 bis 4 Kohlenstoffatomen im Alkoxyteil, für einen jeweils gegebenenfalls einfach bis dreifach, gleich oder verschieden substituierten Phenyl-, Phenoxy-, Phenylthio-, Phenylmethoxy- oder Phenylmethylthiorest, wobei jeweils als Phenylsubstituenten genannt seien: Fluor, Chlor, Brom, Methyl, Ethyl, Hydroxy, Methoxy, Methylthio, Trifluormethyl, Trifluormethoxy, Trifluormethylthio, Dimethylamino, Hydroxycarbonyl, Methoxycarbonyl, Ethoxycarbonyl, Methylcarbonyl, Ethylcarbonyl, Methylcarbonyloxy, Ethylcarbonyloxy, Nitro und Cyano; oder für einen jeweils gegebenenfalls einfach oder zweifach, gleich oder verschieden durch Fluor, Chlor, Brom, Methyl oder Ethyl substituierten Pyridinyl-, Pyrimidinyl-, Furyl- oder Thiophenylrest steht;

Het für einen jeweils gegebenenfalls einfach bis dreifach, gleich oder verschieden substituierten Dioxolanyl- oder Dioxanylrest steht, wobei als Substituenten Methyl, Ethyl, n-Propyl, i-Propyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl in Frage kommen;

n eine der Zahlen 0, 1 oder 2 bedeutet,

m für 0 oder 1 steht; und

$R^2$ für Wasserstoff, einen geradkettigen oder verzweigten Alkylrest mit 1 bis 4 Kohlenstoffatomen, Allyl, Propargyl, Methylcarbonyl, Ethylcarbonyl, Methoxycarbonyl, Ethoxycarbonyl, ferner für einen jeweils gegebenenfalls einfach bis dreifach, gleich oder verschieden substituierten Phenyl- oder Benzylrest, wobei als Substituenten die bei Y bereits genannten Phenylsubstituenten infrage kommen, oder für einen jeweils gegebenenfalls einfach oder zweifach, gleich oder verschieden durch Fluor, Chlor, Brom, Methyl oder Ethyl substituierten Pyridinyl-, Pyrimidinyl-, Furyl- oder Thiophenylrest steht.

Erfindungsgemäß besonders bevorzugt sind diejenigen Verbindungen der allgemeinen Formel (I), in denen

$R^1$ für einen geradkettigen oder verzweigten Alkylrest mit 3 bis 4 Kohlenstoffatomen; für einen jeweils einfach oder mehrfach, gleich oder verschieden substituierten Phenoxymethyl- oder Phenoxyethylrest, wobei als Phenylsubstituenten die bei Y genannten Phenylsubstituenten infrage kommen; oder für eine der Gruppierungen

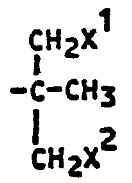 , 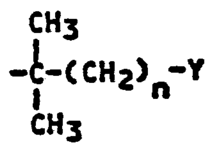 oder 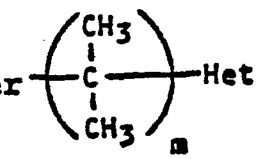

steht, wobei

$X^1$ und $X^2$ ein Fluor- oder Chloratom bedeuten;

Y für Fluor, Chlor, einen geradkettigen oder verzweigten Alkoxy- oder Alkylthiorest mit jeweils 1 bis 2 Kohlenstoffatomen, einen Trifluormethoxy- oder Trifluormethylthiorest, für einen Hydroximinomethyl- oder Alkoximinomethylrest mit 1 bis 2 Kohlenstoffatomen im Alkylteil, für einen 1-Hydroximinoethyl- oder 1-Alkoxyiminoethylrest mit 1 bis 2 Kohlenstoffatomen im Alkoxyteil, für einen jeweils gegebenenfalls einfach bis dreifach, gleich oder verschieden substituierten Phenyl-, Phenoxy-, Phenylthio-, Phenylmethoxy- oder Phenylmethylthiorest, wobei jeweils als Phenylsubstituenten genannt seien: Fluor, Chlor, Brom, Methyl, Hydroxy, Methoxy, Methylthio, Trifluormethyl, Trifluormethoxy, Trifluormethylthio und Hydroxycarbonyl,

Het für einen jeweils gegebenenfalls einfach bis dreifach, gleich oder verschieden substituierten Dioxolanyl- oder Dioxanylrest steht, wobei als Substituenten Methyl, Ethyl, n-Propyl, i-Propyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl in Frage kommen;

n eine der Zahlen 0, 1 oder 2 bedeutet,

m für 0 oder 1 steht; und

$R^2$ für Wasserstoff, einen geradkettigen oder verzweigten Alkylrest mit 1 bis 2 Kohlenstoffatomen, Allyl, Propargyl, Methylcarbonyl, Methoxycarbonyl, ferner für einen jeweils gegebenenfalls einfach bis dreifach, gleich oder verschieden substituierten Phenyl- oder Benzylrest, wobei als Substituenten die bei Y bereits genannten Phenylsubstituenten infrage kommen.

Die erfindungsgemäßen Verbindungen der Formel (I) können auch in der isomeren Form der allgemeinen Formel (Ia)

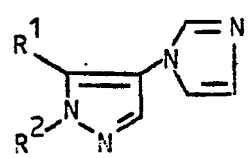

(Ia)

auftreten. Beide isomeren Formen werden erfindungsgemäß beansprucht.

Die erfindungsgemäßen 4-Imidazolylpyrazole der Formel (I) können hergestellt werden, indem man entweder

a) Imidazolyl-Ketoenamine der Formel (II),

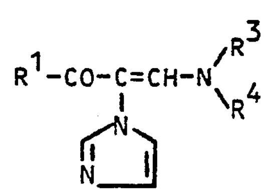

(II)

in welcher

$R^1$ die oben angegebene Bedeutung hat und

$R^3$ und $R^4$ gleich oder verschieden sind und für Alkylreste mit 1 bis 4 Kohlenstoffatomen stehen; oder gemeinsam mit dem N-Atom, an das sie gebunden sind, einen jeweils gegebenenfalls einfach bis dreifach durch Alkylreste mit 1 bis 4 Kohlenstoffatomen substituierten Piperidinyl-, Pyrrolidinyl- oder Morpholinylrest bilden,

mit Hydrazinen der Formel (III),

$$R^2\text{-NH-NH}_2 \qquad \text{(III)}$$

in welcher

$R^2$ die oben angegebene Bedeutung hat,

gegebenenfalls in Gegenwart eines Verdünnungsmittels umsetzt, oder

b) gegebenenfalls die nach dem Verfahren (a) erhältlichen Imidazolyl-pyrazol-Derivate der Formel (Ib),

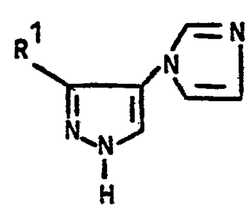

(Ib)

in welcher

$R^1$ die oben angegebene Bedeutung hat,

mit Halogeniden der Formel (IV)

$$Hal\text{-}R^5 \qquad (IV)$$

in welcher

$R^5$ für die Bedeutungen von $R^2$, außer für Wasserstoff, steht und

Hal ein Halogenatom bedeutet,

in Gegenwart einer Base und gegebenenfalls in Gegenwart eines Verdünnungsmittels umsetzt.

An die so erhaltenen Verbindungen der Formel (I) kann gegebenenfalls anschließend eine Säure addiert werden.

Überraschenderweise besitzen die Verbindungen der allgemeinen Formel (I) eine stärkere und spezifischere Hemmwirkung auf die Thromboxansynthese als die aus dem Stand der Technik bekannten Stoffe. Darüber hinaus hemmen die Verbindungen der Formel (I) die Thrombozytenaggregation. Ihre Verwendung in Arzneimitteln mit thromboxansynthetasehemmender Wirkung stellt eine Bereicherung der Pharmazie dar.

Im einzelnen seien außer den Verbindungen der Herstellungsbeispiele die folgenden Verbindungen der allgemeinen Formel (I) genannt, bei welchen $R^2$ für Wasserstoff steht (angegeben ist jeweils die Bedeutung des Restes $R^1$):

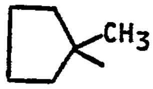

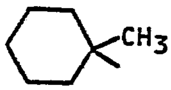

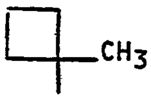

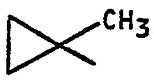

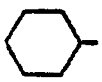

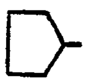

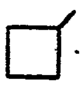

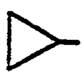

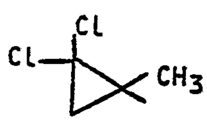

$CH_2=CH-CH_2-C(CH_3)_2-$

$CH\equiv C-CH_2-C(CH_3)_2-$

$NC-CH_2-C(CH_3)_2-$

$(CH_3)_2CH-$

$(CH_3)_2CH-C(CH_3)_2-$

$n-C_3H_7-C(CH_3)_2-$

$n-C_4H_9-C(CH_3)_2-$

$(CH_3)_3C-CH_2-C(CH_3)_2-$

$CH_2=CH-C(CH_3)_2-$

$HO-CH_2-C(CH_3)_2-$

$Cl-CH_2-C(CH_3)_2-$

$F-CH_2-C(CH_3)_2-$

$CH_3-C(CH_2Cl)_2-$

$Cl-CH_2CH_2-C(CH_3)_2-$

$F-CH_2CH_2-C(CH_3)_2-$

$CH_3CO-O-CH_2-C(CH_3)_2-$

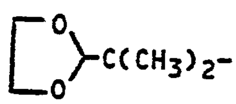

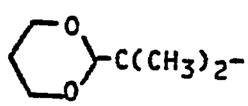

$CF_3S-C(CH_3)_2-$

$CH_3O-N=CH-C(CH_3)_2-$

$n-C_4H_9O-CH_2-C(CH_3)_2-$

$C_2H_5CO-O-CH_2-C(CH_3)_2-$

$i-C_3H_7CO-O-CH_2-C(CH_3)_2-$

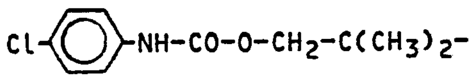

$(CH_3)_2N-CO-O-CH_2-C(CH_3)_2-$

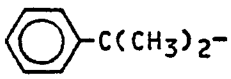

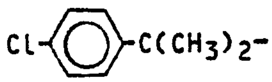

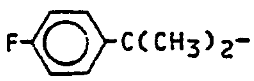

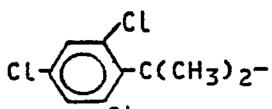

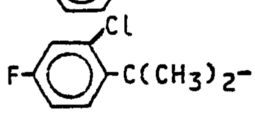

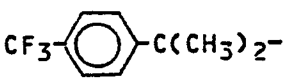

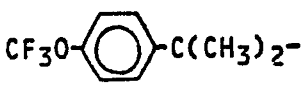

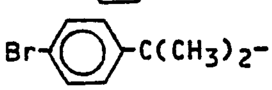

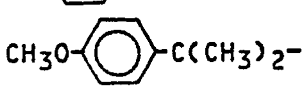

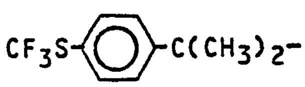

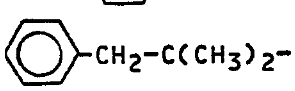

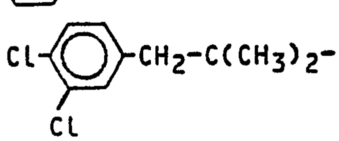

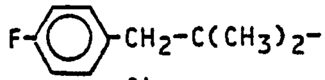

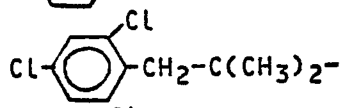

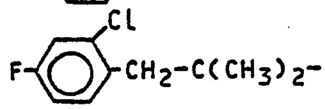

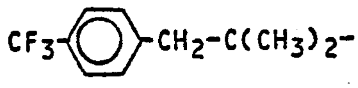

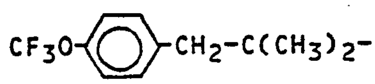

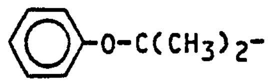

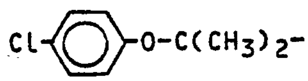

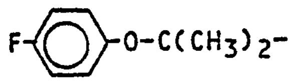

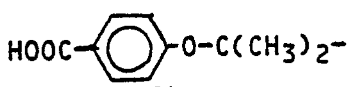

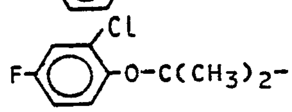

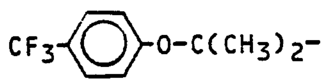

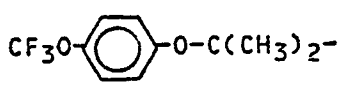

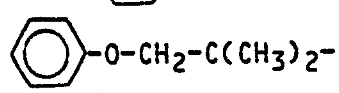

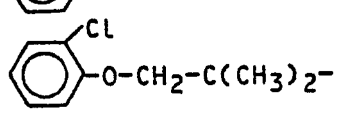

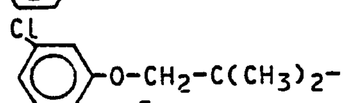

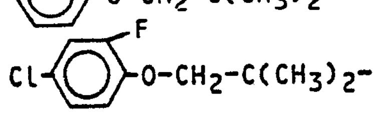

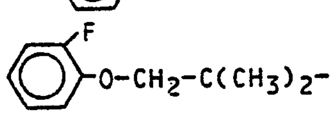

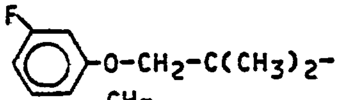

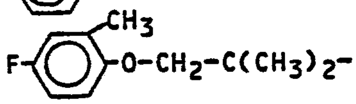

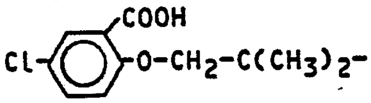

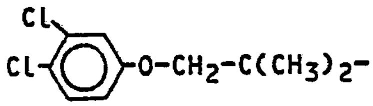

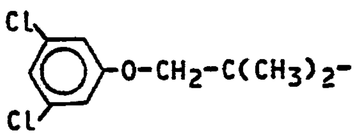

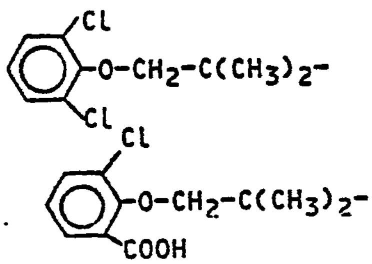

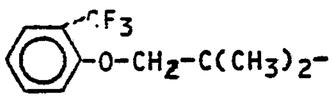

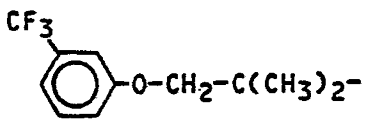

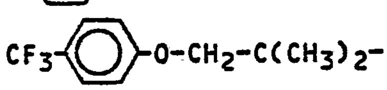

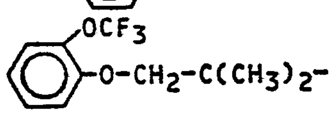

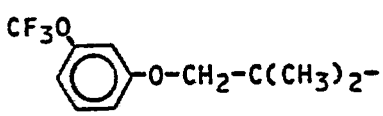

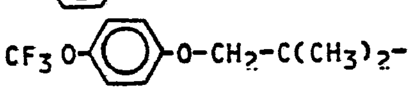

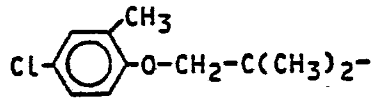

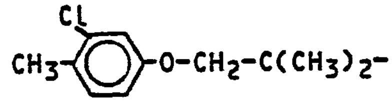

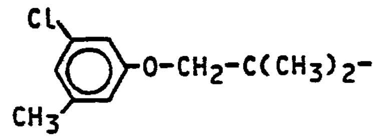

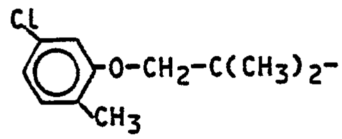

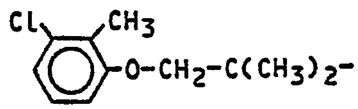

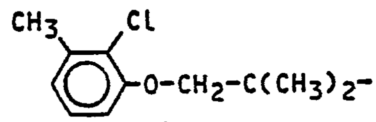

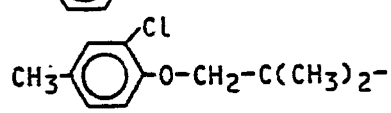

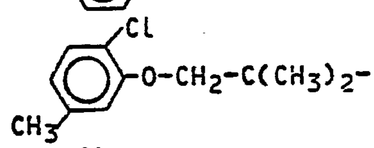

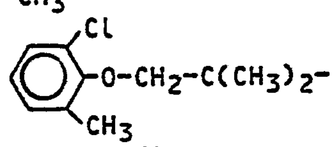

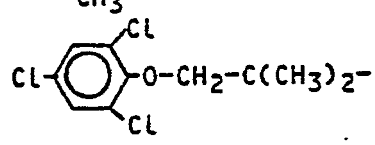

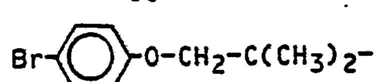

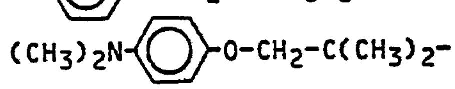

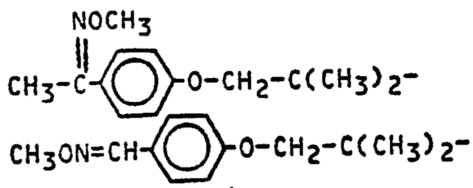

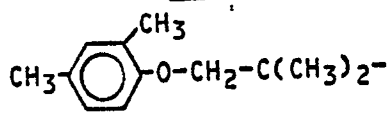

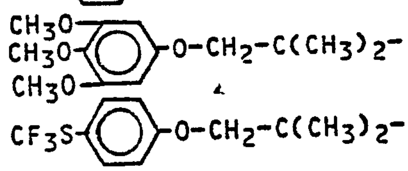

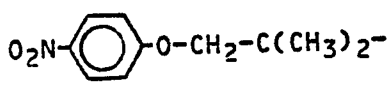

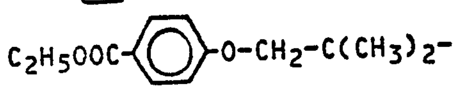

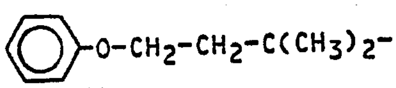

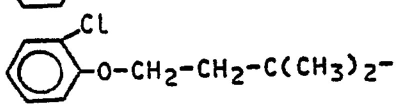

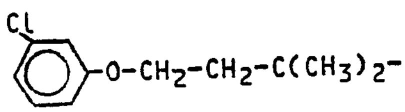

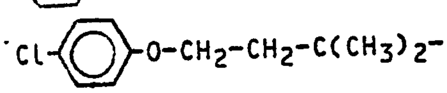

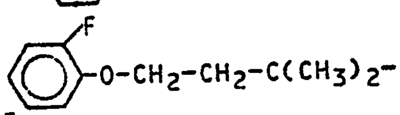

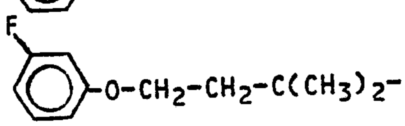

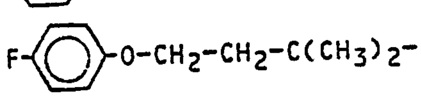

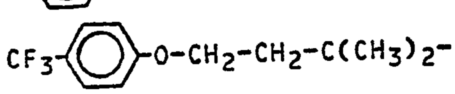

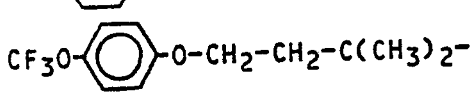

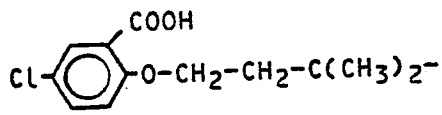

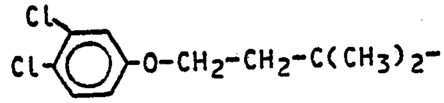

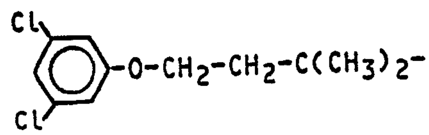

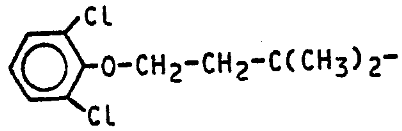

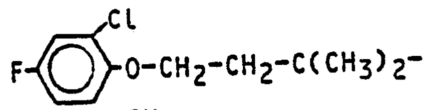

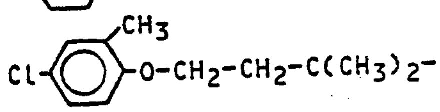

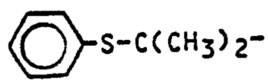

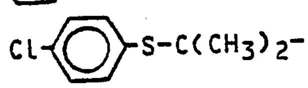

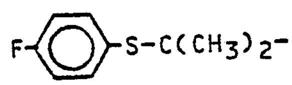

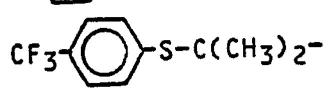

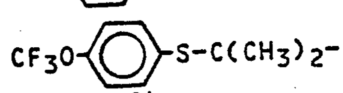

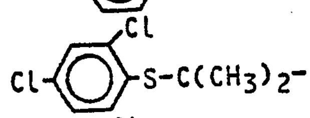

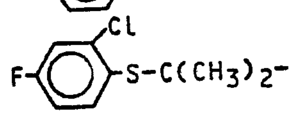

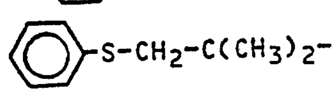

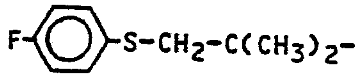
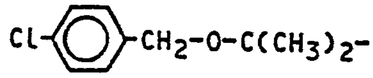
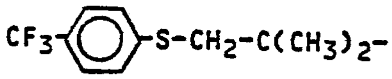
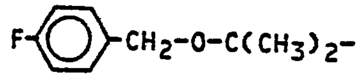
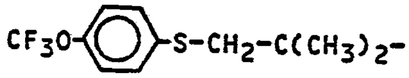
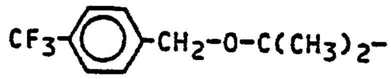
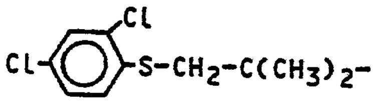
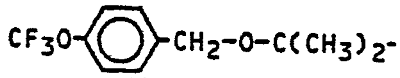
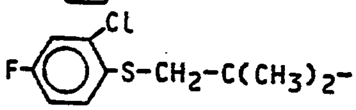
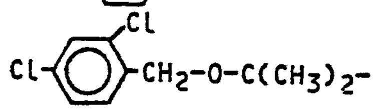
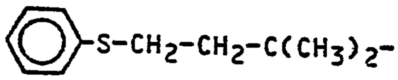
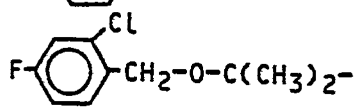
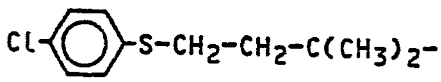
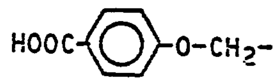
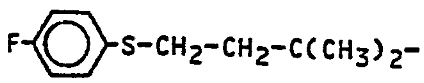
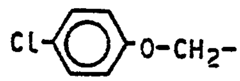
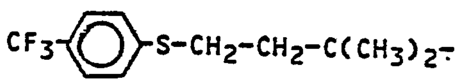
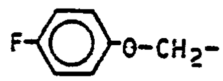
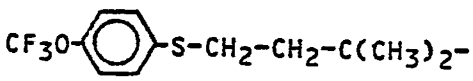
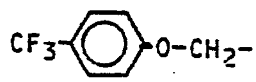
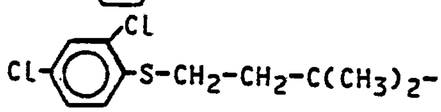
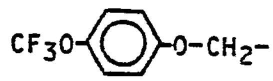
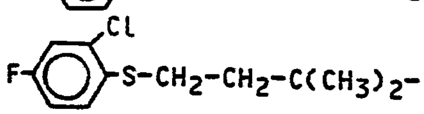
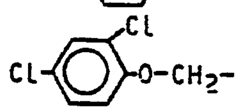
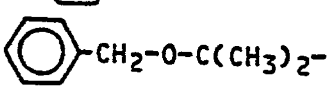
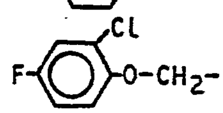

In der nachfolgenden Tabelle sind erfindungsgemäße Verbindungen aufgeführt, bei denen $R^2 \neq H$:

| $R^1$ | $R^2$ |
| --- | --- |
| 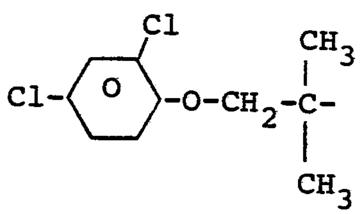 | $-CH_3$ |
| " | $-COCH_3$ |

| $R^1$ | $R^2$ |
| --- | --- |
| 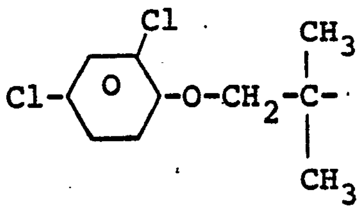 | $-CH_2-CH=CH_2$ |
| " | 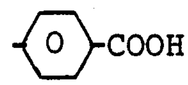 |
| 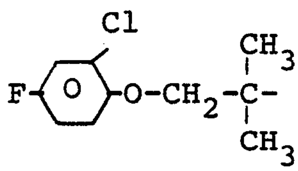 | $-CH_3$ |
| " | $-COOCH_3$ |
| " | 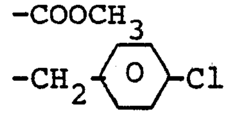 |
| " | 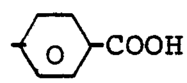 |
| 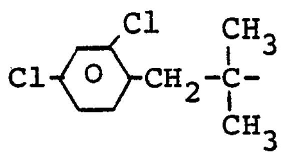 | $-CH_3$ |
| " | $-COCH_3$ |
| " | $-CH_2-C\equiv CH$ |
| " | 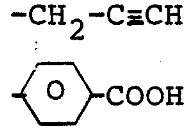 |

Nachstehend seien die Verfahren zur Herstellung der erfindungsgemäßen Verbindungen näher erläutert.

Verwendet man beispielsweise 1-Dimethylamino-4,4-dimethyl-2-(imidazol-1-yl)-1-penten-3-on und Hydrazin als Ausgangsstoffe, so kann der Ablauf des erfindungsgemäßen Verfahrens (a) durch das folgende Formelschema wiedergegeben werden:

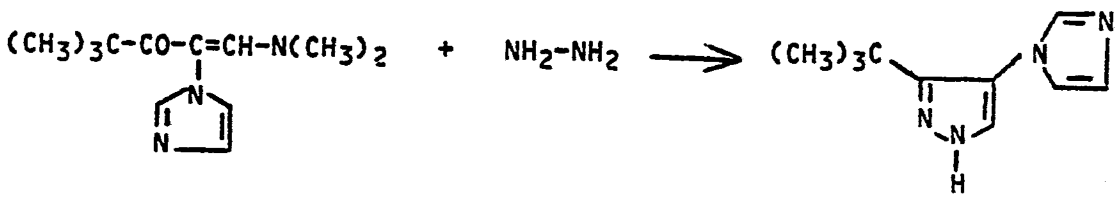

Verwendet man beispielsweise 1-Dimethylamino-4,4-dimethyl-2-(imidazol-1-yl)-1-penten-3-on und Methylhydrazin als Ausgangsstoffe, so kann der Ablauf des erfindungsgemäßen Verfahrens (a) durch das folgende Formelschema wiedergegeben werden:

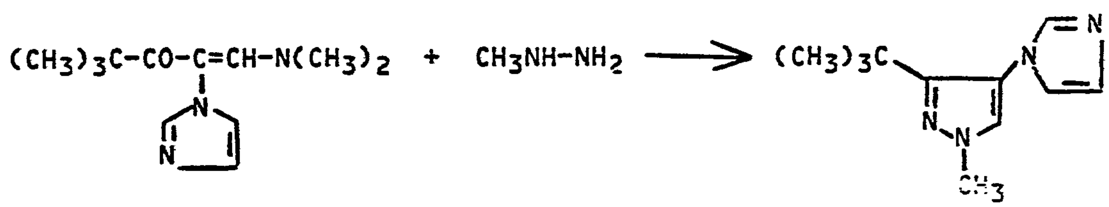

Verwendet man beispielsweise 3-tert.-Butyl-4-(imidazol-1-yl)-pyrazol und Acetylchlorid als Ausgangsstoffe, so kann der Ablauf des erfindungsgemäßen Verfahrens (b) durch das folgende Formelschema wiedergegeben werden:

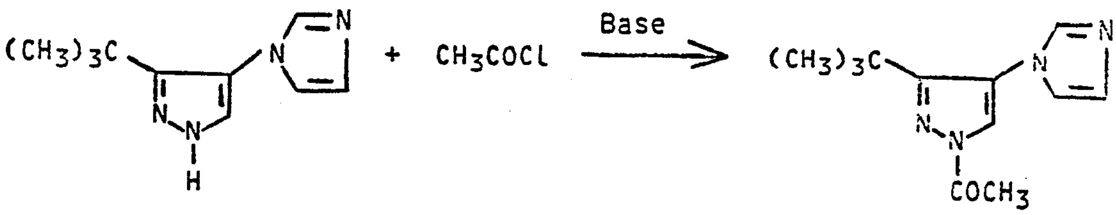


Die im erfindungsgemäßen Verfahren (a) als Ausgangsstoffe zu verwendenden Imidazolyl-Ketoenamine sind durch die obige Formel (II) allgemein definiert.

Die Imidazolyl-Ketoenamine der Formel (II) sind bekannt (vgl. DE-OS 3 000 643 bzw. die deutsche Patentanmeldung P 32 29275 vom 04.08.1982) und können erhalten werden, indem man Imidazolylketone der Formel (V),

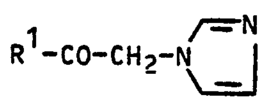

(V)

in welcher

$R^1$ die oben angegebene Bedeutung hat,

mit Amidacetalen bzw. Aminalestern der Formeln

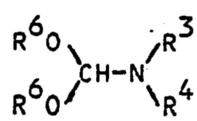

(VIa)

beziehungsweise

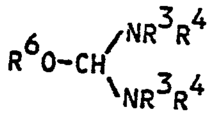

(VIb)

in welcher

$R^3$ und $R^4$ die oben angegebenen Bedeutungen haben und

$R^6$ für Alkyl mit 1 bis 4 Kohlenstoffatomen steht,

in an sich bekannter Art und Weise in Gegenwart eines inerten organischen Lösungsmittels, wie beispielsweise eines aromatischen Kohlenwasserstoffes, in der Siedehitze umsetzt, wobei man vorzugsweise Amidacetal bzw. Aminalester der Formel (VIa) bzw. (VIb) im Überschuß einsetzt (vgl. hierzu auch Chem. Ber. <u>101</u>, 41 - 50 (1968); J. Org. Chem. <u>43</u>, 4248 - 4250 (1978) sowie die Herstellungsbeispiele).

Die Imidazolylketone der Formel (V) sind bekannt (vgl. beispielsweise DE-OS 3 048 266 und DE-OS 31 45 858) bzw. können sie nach üblichen Methoden hergestellt werden, indem man z.B. die entsprechenden Halogen-Ketone in Gegenwart eines Säurebinders mit Imidazol umsetzt.

Die außerdem für das erfindungsgemäße Verfahren (a) als Ausgangsstoffe zu verwendenden Hydrazine sind durch die Formel (III) allgemein definiert. Die Hydrazine der Formel (III) sind allgemein bekannte Verbindungen der organischen Chemie.

Die im erfindungsgemäßen Verfahren (b) als Ausgangsstoffe zu verwendenden Derivate der Formel (Ib) sind erfindungsgemäße Stoffe.

Die außerdem für das erfindungsgemäße Verfahren (b) als Ausgangsstoffe zu verwendenden Halogenide sind durch die Formel (IV) allgemein definiert.

Auch die Halogenide der Formel (IV) sind allgemein bekannte Verbindungen der organischen Chemie.

Als Verdünnungsmittel kommen für das erfindungsgemäße Verfahren (a) unter den Reaktionsbedingungen inerte organische Lösungsmittel infrage. Hierzu gehören vorzugsweise Ether, wie z.B. Diethylether, Diisopropylether, Dimethoxyethan, Dioxan oder Tetrahydrofuran; Nitrile, wie z.B. Acetonitril oder Propionitril; aromatische Kohlenwasserstoffe, wie z.B. Benzol, Toluol oder Chlorbenzol; aliphatische Kohlenwasserstoffe, wie z.B. Cyclohexan oder 1,2-Dichlorethan; Amide, wie z.B. Dimethylformamid oder Dimethylacetamid; Sulfoxide, wie z.B. Dimethylsulfoxid; sowie Hexamethylphosphorsäuretriamid.

Die Reaktionstemperaturen können beim erfindungsgemäßen Verfahren (a) in einem größeren Bereich variiert werden. Im allgemeinen arbeitet man zwischen 0 und 150°C, vorzugsweise zwischen 10 und 100°C.

Bei der Durchführung des erfindungsgemäßen Verfahrens (a) setzt man in der Regel auf 1 Mol Imidazolyl-Ketoenamin der Formel (II) 1 bis 2 Mol, vorzugsweise 1 bis 1,1 Mol Hydrazin der Formel (III) ein. Die Isolierung der Endprodukte erfolgt in üblicher Weise.

Als Verdünnungsmittel kommen für das erfindungsgemäße Verfahren (b) alle unter den Reaktionsbedingungen inerten organischen Lösungsmittel infrage. Hierzu gehören vorzugsweise Ether, wie z.B. Diethylether, Dimethoxymethan, Tetrahydrofuran oder Dioxan; Nitrile, wie z.B. Acetonitril oder Propionitril; Amide, wie z.B. Dimethylformamid oder Dimethylacetamid; Sulfoxide, wie z.B. Dimethylsulfoxid; sowie Hexamethylphosphorsäuretriamid.

Das erfindungsgemäße Verfahren (b) wird in Gegenwart einer Base durchgeführt. Geeignete Basen sind beispielsweise Alkalihydroxide, wie Natriumhydroxid oder Kaliumhydroxid; Alkalialkoholate, wie Natriummethylat oder Kalium-tert.-butylat; Alkalihydride, wie Natriumhydrid; sowie metallorganische Verbindungen, wie Butyllithium, tert.-Butyllithium oder Phenyllithium.

Die Reaktionstemperaturen können beim erfindungsgemäßen Verfahren (b) in einem größeren Bereich variiert werden. Im allgemeinen arbeitet man zwischen 0 und 150°C, vorzugsweise zwischen 10 und 100°C.

0115640

Bei der Durchführung des erfindungsgemäßen Verfahrens (b) setzt man in der Regel auf 1 Mol Imidazolyl-pyrazol-Derivat der Formel (Ia) 1 bis 2 Mol, vorzugsweise 1 bis 1,2 Mol an Halogenid der Formel (IV) ein. Die Isolierung der Endprodukte erfolgt in üblicher Weise.

Die nach den erfindungsgemäßen Verfahren erhältlichen Verbindungen der Formel (I) können in Säureadditions-Salze überführt werden.

Zur Herstellung von physiologisch verträglichen Säureadditions-Salzen der Verbindungen der Formel (I) kommen vorzugsweise folgende Säuren infrage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, Isolieren und gegebenenfalls durch Waschen mit einem inerten organischen Lösungsmittel gereinigt werden.

Bei ihrer pharmazeutischen Anwendung können die erfindungsgemäßen Verbindungen als solche der in Form ihrer physiologisch unbedenklichen Salze eingesetzt werden.

Als Zubereitungsformen kommen die üblichen galenischen Applikationsformen infrage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und/oder Trägerstoffe.

Der Einsatz der so hergestellten Arzneimittel erfolgt je nach Bedarf z.B. durch lokale, parenterale oder orale Verabreichung.

Besonders geeignet sind Formulierungen, die die erfindungsgemäßen Verbindungen in einer Konzentration von etwa 0,1 bis 10 Gew.-% enthalten. Besonders bevorzugt sind wäßrige Lösungen, die gegebenenfalls auf einen pH-Wert von 6 bis 2 gepuffert sind.

Die Dosierung der erfindungsgemäßen Verbindungen liegt vorzugsweise in einem Bereich von 0,05 bis 100 mg/kg, insbesondere von 0,1 bis 20 mg/kg Körpergewicht.

Herstellungsbeispiele

Beispiel 1

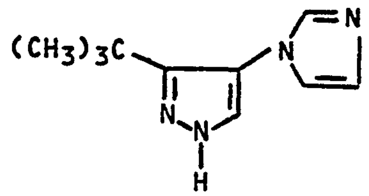

(Verfahren a)

22,1 g (0,1 Mol) 1-Dimethylamino-4,4-dimethyl-2-(imidazol-1-yl)-1-penten-3-on und 10 g (0,2 Mol) Hydrazinhydrat werden 15 Minuten unter Rückfluß erhitzt. Man läßt abkühlen und versetzt das Reaktionsgemisch mit Wasser. Der wasserunlösliche Rückstand wird abgetrennt und in Diethylether verrührt. Der kristalline Rückstand wird abgesaugt und bei 60°C im Vakuum getrocknet. Man erhält 10,5 g (55,3 % der Theorie) 4-tert.-Butyl-4-(imidazol-1-yl)-pyrazol vom Schmelzpunkt 165°C.

Herstellung des Ausgangsproduktes

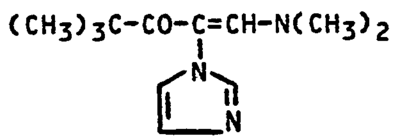

41,6 g (0,25 Mol) 3,3-Dimethyl-1-(imidazol-1-yl)-butan-2-on werden mit 35,7 g (0,3 Mol) Dimethylformamid-dimethylacetal 5 Stunden unter Rückfluß erhitzt. Danach

wird das überschüssige Acetal abdestilliert. Das zurückbleibende Öl kristallisiert beim Abkühlen. Man erhält 50 g (90,5 % der Theorie) 4,4-Dimethyl-1-dimethylamino-2-(imidazol-1-yl)-1-penten-3-on vom Schmelzpunkt 45 bis 50°C.

Beispiel 2

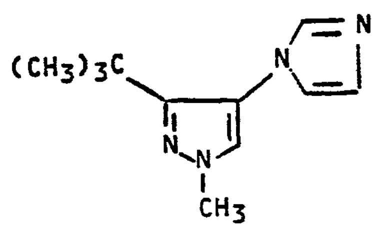

(Verfahren a)

22,1 g (0,1 Mol) 1-Dimethylamino-4,4-dimethyl-2-(imidazol-1-yl)-1-penten-3-on und 4,6 g (0,1 Mol) Methylhydrazin werden bei 80°C gerührt. Man läßt abkühlen und verrührt das Reaktionsgemisch mit Wasser. Der kristalline Niederschlag wird abfiltriert, mit Wasser gewaschen und im Vakuum bei 60°C getrocknet.

Man erhält 10,4 g (51 % der Theorie) 3-tert.-Butyl-1-methyl-4-(imidazol-1-yl)-pyrazol vom Schmelzpunkt 74 bis 76°C.

Beispiel 3

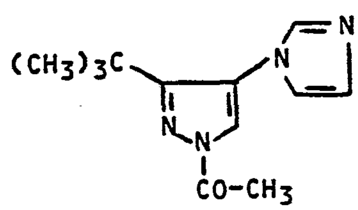

(Verfahren b)

11,4 g (0,06 Mol) 3-tert.-Butyl-4-(imidazol-1-yl)-pyrazol (Beispiel 1) werden bei Raumtemperatur zu einer Suspension von 1,44 g (0,06 Mol) Natriumhydrid in 100 ml Tetrahydrofuran gegeben. Man rührt das Reaktionsgemisch bis zur Beendigung der Wasserstoffentweichung nach und versetzt dann mit 4,95 g (0,063 Mol) Acetylchlorid. Man läßt 4 Stunden unter Rückfluß rühren, kühlt ab und gießt auf Wasser. Danach wird mit Methylenchlorid extrahiert. Die vereinigten Methylenchloridphasen werden mit Natriumhydrogencarbonat und Wasser gewaschen, über Natriumsulfat getrocknet und eingeengt. Der Rückstand kristallisiert beim Verrühren in Petrolether. Die Kristalle werden abgesaugt und im Vakuum bei 60°C getrocknet. Man erhält 9,9 g (71,1 % der Theorie) 1-Acetyl-3-tert.-butyl-4-(imidazol-1-yl)-pyrazol vom Schmelzpunkt 80°C.

In analoger Weise und entsprechend den erfindungsgemäßen Verfahren werden die Verbindungen der allgemeinen Formel (I)

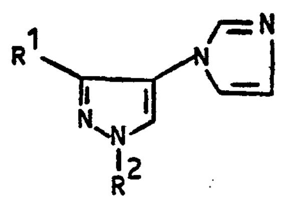

(I)

der nachfolgenden Tabelle 2 erhalten:

| Beisp.-Nr. | $R^1$ | $R^2$ | Schmp. °C bzw. $n_D^{20}$ |
|---|---|---|---|
| 4 | $(CH_3)_3C-$ | 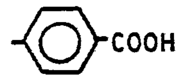 | 174 |
| 5 | 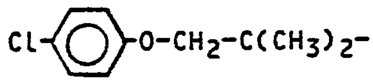 | H | 143 |
| 6 | $C_2H_5O-CH_2-C(CH_3)_2-$ | H | 84 |
| 7 | 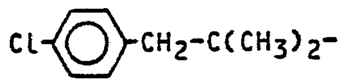 | H | 158 |
| 8 | 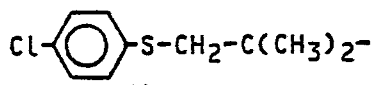 | H | 108 |
| 9 | 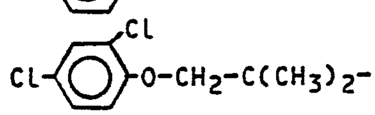 | H | 140-141 |
| 10 | $CH_3-C(CH_2F)_2-$ | H | 158-160 |
| 11 | 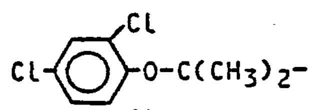 | H | 148 |
| 12 | 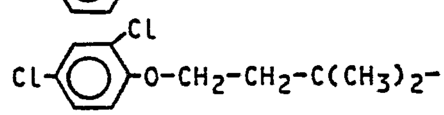 | H | 48 |
| 13 | 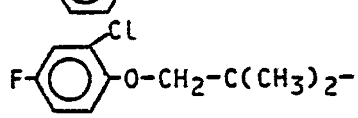 | H | 134 |
| 14 | 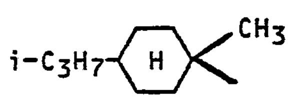 | H | 103 |
| 15 | 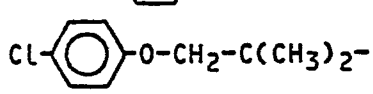 | $-CH_3$ | 1,5644 |
| 16 | 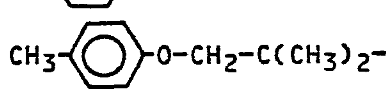 | H | 122 |
| 17 | 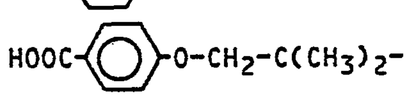 | H | 254 |
| 18 | 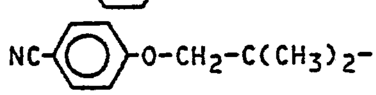 | H | 160 |
| 19 | 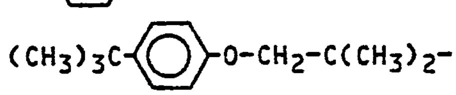 | H | 156-158 |

| Beisp.-Nr. | $R^1$ | $R^2$ | Schmp. °C bzw. $n_D^{20}$ |
|---|---|---|---|
| 20 | 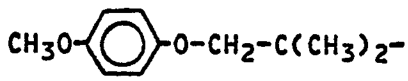 | H | zähes Oel |
| 21 | 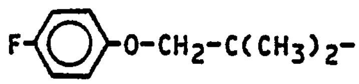 | H | 133 |
| 22 | 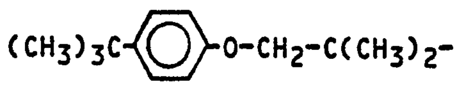 | 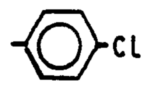 | 98 |
| 23 | 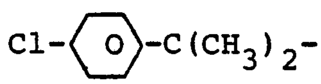 | H | 181 |
| 24 | 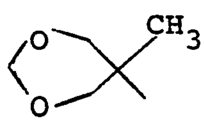 | H | 190 |
| 25 | 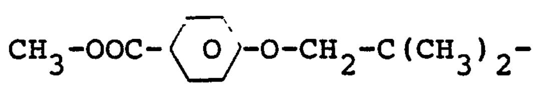 | H | 154 |

Verwendungsbeispiele

Beispiel A

Thrombozytenaggregationshemmung

Für die Bestimmung der thrombozytenaggregationshemmenden Wirkung wurde Blut von gesunden Spendern, die mindestens 14 Tage lang kein Medikament eingenommen hatten, verwendet. Das Blut wurde in 3,8 %-iger Natriumcitratlösung aufgenommen. Plättchenreiches Plasma (PRP) wurde durch 20 Minuten Zentrifugation bei 150 g und Raumtemperatur gewonnen (Jürgens/Beller: Klinische Methoden der Blutgerinnungsanalyse; Thieme-Verlag Stuttgart 1959). Die Plättchenaggregation wurde nach der turbidometrischen Methode (Born, G.V.R.: J. Physiol. 162, 67 (1962)) im Aggregometer bei 37°C bestimmt. Hierzu wurde PRP mit Prüfsubstanz bei 37°C inkubiert und anschließend die Aggregation durch Zugabe einer Kollagensuspension ausgelöst. Bestimmt wurde die minimale Konzentration einer Prüfsubstanz, die die Thrombozytenaggregation hemmte.

In diesem Test wird die Thrombozytenaggregation insbesondere durch die Verbindungen folgender Herstellungsbeispiele gehemmt: 1, 2, 9 und 15.

Beispiel B

Thromboxansynthetasehemmung

Der Arachidonsäuremetabolismus in Human-Thrombozyten wurde mit Hilfe von Tritium-markierter Arachidonsäure untersucht. Die Thrombozyten metabolisieren die Arachidonsäure über den Cyclooxygenaseweg vor allem zu $TXA_2$ und HHT und über den Lipoxygenaseweg zu 12-HETE, die dünnschichtchromatographisch getrennt werden können (Bailey, J.M. et al., Prostaglandins 13, 479 - 492 (1977)). Inhibitoren der einzelnen enzymatischen Reaktionen verändern das chromatographische Verteilungsmuster in charakteristischer Weise.

Gewaschene Human-Thrombozyten von gesunden Spendern, die mindestens seit 14 Tagen kein Medikament eingenommen hatten, wurden mit Prüfsubstanz 2 Minuten bei 37°C inkubiert und anschließend mit $^3H$-Arachidonsäure weitere 10 Minuten bei 37°C inkubiert. Die Suspension wurde angesäuert und mit Essigester extrahiert. Der Essigester wurde unter $N_2$ abgedampft und der Rückstand in $CH_3OH/CHCl_3$ (1:1) aufgenommen und auf DC-Plastikfolien aufgetragen. Die Trennung erfolgte mit einem Fließmittelgemisch $CHCl_3/CH_3OH$/Eisessig/$H_2O$ (80 : 8 : 1 : 0,8). Die Verteilung der Radioaktivität wurde mittels eines Radioscanners gemessen. Bestimmt wurde die minimale Konzentration einer Prüfsubstanz, die zu signifikanten Veränderungen des Verteilungsmusters der Eikosanoide führte.

In diesem Test wird die $TXA_2$-Synthese insbesondere durch die Verbindungen folgender Herstellungsbeispiele gehemmt, ohne daß die Cyclooxygenase signifikant beeinflußt wird: 1, 2, 9, 10 und 15.

Tabelle zu Beispielen A und B

| Substanz gemäß Beispiel-Nr. | $TXA_2$-Synthetasehemmung | Hemmung der Thrombozytenaggregation |
|---|---|---|
| | minimal effektive Konzentration (g/ml) | |
| 1 | $3-1x10^{-6}$ | $3-1x10^{-5}$ |
| 2 | $1x10^{-6}-3x10^{-7}$ | $3-1x10^{-5}$ |
| 10 | $3-1x10^{-6}$ | -- |
| 15 | $1x10^{-6}-3x10^{-7}$ | $3x10^{-5}$ |
| 9 | $1x10^{-4}-3x10^{-5}$ | $3-1x10^{-5}$ |
| Imidazol[x)] | $1x10^{-4}-3x10^{-5}$ | $3x10^{-5}$ |

x) Imidazol ist ein spezifischer $TXA_2$-Synthetasehemmer (Needleman, P., Raz, A., Ferrendelli, J.A., Minkes, M.: Proc. Natl. Acad. Sci. USA 74, 1716 - 1720, (1977)).

Patentansprüche

1. Verbindungen der allgemeinen Formel (I)

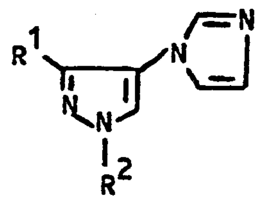 (I)

in welcher

$R^1$ für einen geradkettigen oder verzweigten Alkylrest mit 1 bis 12 C-Atomen; für einen gegebenenfalls einfach oder mehrfach, gleich oder verschieden durch Halogen oder $C_1$-$C_4$-Alkyl- oder Alkoxyreste substituierten Cycloalkylrest mit 3 bis 7 C-Atomen oder Cycloalkylalkylrest mit 3 bis 7 C-Atomen im Cycloalkylteil und 1 bis 4 C-Atomen im Alkylteil; für einen gegebenenfalls einfach oder mehrfach, gleich oder verschieden durch einen der bei Y genannten Phenylsubstituenten substituierten Phenoxyalkylrest mit 1 bis 4 Kohlenstoffatomen im Alkylteil; oder für eine der Gruppierungen

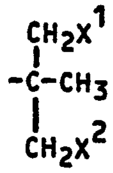 , 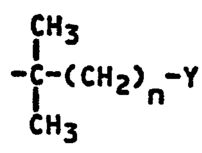 oder 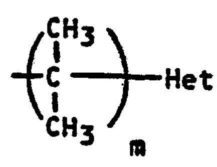 steht,

wobei

$X^1$ und $X^2$ Halogenatome bedeuten,

Y für Halogen; für Hydroxy; für einen Alkoxy- oder Alkylthiorest mit jeweils 1 bis 4 Kohlenstoffatomen; für einen Halogenalkoxy- oder Halogenalkylthiorest mit jeweils 1 bis 2 Kohlenstoffatomen und 1 bis 5 gleichen oder verschiedenen Halogenatomen; für einen Alkenyl- oder Alkinylrest mit jeweils 2 bis 6 Kohlenstoffatomen; für einen Alkoxycarbonyl- oder Alkylcarbonyloxyrest mit jeweils 1 bis 4 Kohlenstoffatomen im Alkylteil; für einen gegebenenfalls einfach oder zweifach, gleich oder verschieden durch Alkyl mit 1 bis 4 Kohlenstoffatomen oder jeweils gegebenenfalls einfach bis dreifach, gleich oder verschieden substituiertes Phenyl oder Phenylalkyl mit 1 bis 2 Kohlenstoffatomen im Alkylteil, wobei jeweils der Phenylrest als Substituenten Halogen, Alkyl mit 1 bis 4 Kohlenstoffatomen oder Trifluormethyl enthalten kann, substituierten Carbamoyloxyrest; für einen Aldoxim- oder Ketoxim-Rest oder deren Ether-Derivate; für einen jeweils gegebenenfalls einfach oder mehrfach, gleich oder verschieden durch Halogen, Alkylreste mit 1 bis 4 Kohlenstoffatomen, Hydroxy, Alkoxy- oder Alkylthioreste mit jeweils 1 bis 2 Kohlenstoffatomen, Halogenalkyl-, Halogenalkoxy- oder Halogenalkylthioreste mit jeweils 1 bis 2 Kohlenstoffatomen und 1 bis 5 gleichen oder verschiedenen Halogenatomen, Dialkylaminorest mit 1 bis 4 Kohlenstoffatomen in jedem

Alkylteil, Hydroxycarbonyl-, Alkoxycarbonyl-, Alkylcarbonyl- oder Alkylcarbonyloxyreste mit jeweils 1 bis 4 Kohlenstoffatomen in jedem Alkylteil, Nitro oder Cyano substituierten Phenyl-, Phenoxy-, Phenylthio-, Phenylalkoxy- oder Phenylalkylthiorest mit jeweils 1 bis 4 C-Atomen im Alkylteil; oder für einen gegebenenfalls einfach oder mehrfach, gleich oder verschieden durch Alkylreste mit 1 bis 2 Kohlenstoffatomen und/oder Halogen substituierten 5- oder 6-gliedrigen Heteroarylrest mit 1 bis 2 Heteroatomen, steht;

Het einen gegebenenfalls einfach oder mehrfach, gleich oder verschieden durch Alkylreste mit 1 bis 4 Kohlenstoffatomen oder durch jeweils gegebenenfalls einfach oder mehrfach, gleich oder verschieden substituierte Phenyl- oder Phenoxyalkylreste mit 1 bis 4 Kohlenstoffatomen im Alkylteil, substituierten 5- oder 6-gliedrigen heteroaliphatischen Rest mit Sauerstoff und/oder Schwefel als Heteroatome darstellt, wobei die Phenylreste durch Halogen, Alkylreste mit 1 bis 4 Kohlenstoffatomen, Alkoxy- oder Alkylthioreste mit jeweils 1 bis 2 Kohlenstoffatomen sowie Halogenalkyl-, Halogenalkoxy- oder Halogenalkylthioreste mit jeweils 1 bis 2 Kohlenstoffatomen und 1 bis 5 gleichen oder verschiedenen Halogenatomen substituiert sein können;

n    eine der Zahlen 0, 1 oder 2 bedeutet und

m    für 0 oder 1 steht; ferner

$R^2$    für Wasserstoff; für ein geradkettigen oder verzweigten Alkylrest mit 1 bis 12 Kohlenstoffatomen; für einen Alkenyl- oder Alkinylrest mit jeweils 3 bis 6 Kohlenstoffatomen; für einen Alkylcarbonyl- oder Alkoxycarbonylrest mit jeweils 1 bis 4 Kohlenstoffatomen im Alkylteil; für einen jeweils gegebenenfalls einfach oder mehrfach, gleich oder verschieden durch die bei Y bereits genannten Phenylsubstituenten substituierten Phenyl- oder Benzylrest; oder für einen gegebenenfalls einfach oder mehrfach, gleich oder verschieden durch Alkylreste mit 1 bis 2 Kohlenstoffatomen und/oder Halogen substituierten 5- oder 6-gliedrigen Heteroarylrest mit 1 bis 2 Heteroatomen steht,

sowie pharmakologisch unbedenkliche Säureadditionssalze von Verbindungen der Formel (I).

2. Verbindungen gemäß Anspruch 1, dadurch gekennzeichnet, daß

$R^1$ für einen geradkettigen oder verzweigten Alkylrest mit 3 bis 8 Kohlenstoffatomen; für einen jeweils gegebenenfalls einfach bis dreifach, gleich oder verschieden durch Chlor, Brom, Methyl, Ethyl, Isopropyl, Methoxy oder Ethoxy substituierten Cycloalkylrest mit 3 bis 7 Kohlenstoffatomen oder Cycloalkylmethylrest mit 3 bis 7 Kohlenstoffatomen im Cycloalkylteil; für einen jeweils einfach oder mehrfach, gleich oder verschieden durch die bei Y genannten Phenylsubstituenten substituierten Phenoxymethyl- oder Phenoxyethylrest; oder für eine der Gruppierungen

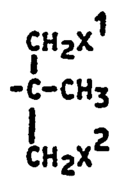 , 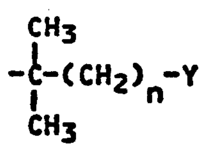 oder 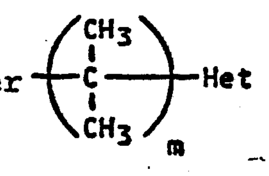

steht, wobei

$X^1$ und $X^2$ ein Fluor-, Chlor- oder Bromatom bedeuten;

Y für Fluor, Chlor, Brom, Hydroxy, einen geradkettigen oder verzweigten Alkoxy- oder Alkylthiorest mit jeweils 1 bis 4 Kohlenstoffatomen, einen Trifluormethoxy-, Trifluormethylthio-, Vinyl-,

Allyl-, Acetylenyl-, Propargyl-, Alkoxycarbonyl oder Alkylcarbonyloxyrest mit jeweils 1 bis 4 Kohlenstoffatomen im Alkylteil, einen Alkyl- oder Dialkylcarbamoyloxyrest mit jeweils 1 bis 2 Kohlenstoffatomen in jedem Alkylteil, einen Phenyl- oder Phenyl-alkyl-carbamoyloxyrest mit 1 bis 2 Kohlenstoffatomen im Alkylteil, wobei der Phenylrest jeweils einfach bis dreifach, gleich oder verschieden durch Fluor, Chlor, Methyl oder Trifluormethyl substituiert sein kann, für einen Hydroximinomethyl- oder Alkoximinomethylrest mit 1 bis 4 Kohlenstoffatomen im Alkoxyteil, für einen 1-Hydroximinoethyl- oder 1-Alkoximinoethylrest mit 1 bis 4 Kohlenstoffatomen im Alkoxyteil, für einen jeweils gegebenenfalls einfach bis dreifach, gleich oder verschieden durch Fluor, Chlor, Brom, Methyl, Ethyl, Hydroxy, Methoxy, Methylthio, Trifluormethyl, Trifluormethoxy, Trifluormethylthio, Dimethylamino, Hydroxycarbonyl, Methoxycarbonyl, Ethoxycarbonyl, Methylcarbonyl, Ethylcarbonyl, Methylcarbonyloxy, Ethylcarbonyloxy, Nitro oder Cyano substituierten Phenyl-, Phenoxy-, Phenylthio-, Phenylmethoxy- oder Phenylmethylthiorest; oder für einen jeweils gegebenenfalls einfach oder zweifach , gleich oder verschieden durch Fluor, Chlor, Brom, Methyl oder Ethyl substituierten Pyridinyl-, Pyrimidinyl-, Furyl- oder Thiophenylrest steht;

Het für einen jeweils gegebenenfalls einfach bis dreifach, gleich oder verschieden durch Methyl, Ethyl, n-Propyl, i-Propyl sowie jeweils gegebenenfalls einfach bis dreifach, gleich oder verschieden durch Fluor, Chlor, Methyl, Trifluormethyl oder Trifluormethoxy substituiertes Phenyl oder Phenoxymethyl substituierten Dioxolanyl- oder Dioxanylrest steht;

n eine der Zahlen 0,1 oder 2 bedeutet,

m für 0 oder 1 steht; und

$R^2$ für Wasserstoff, einen geradkettigen oder verzweigten Alkylrest mit 1 bis 4 Kohlenstoffatomen, Allyl, Propargyl, Methylcarbonyl, Ethylcarbonyl, Methoxycarbonyl, Ethoxycarbonyl, ferner für einen jeweils gegebenenfalls einfach bis dreifach, gleich oder verschieden durch einen der bei Y bereits genannten Phenylsubstituenten substituierten Phenyl- oder Benzylrest, oder für einen jeweils gegebenenfalls einfach oder zweifach, gleich oder verschieden durch Fluor,

Chlor, Brom, Methyl und Ethyl substituierten Pyridinyl-, Pyrimidinyl-, Furyl- oder Thiophenylrest steht.

3. Verbindungen gemäß Ansprüchen 1 und 2, dadurch gekennzeichnet, daß

$R^1$ für einen geradkettigen oder verzweigten Alkylrest mit 3 bis 4 Kohlenstoffatomen; für einen jeweils einfach oder mehrfach, gleich oder verschieden substituierten Phenoxymethyl- oder Phenoxyethylrest, wobei als Phenylsubstituenten die bei Y genannten Phenylsubstituenten infrage kommen; oder für eine der Gruppierungen

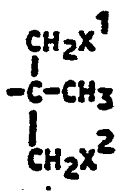 , 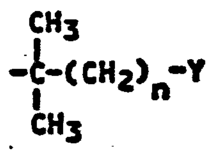 oder 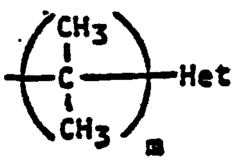

steht, wobei

$X^1$ und $X^2$ ein Fluor- oder Chloratom bedeuten;

Y für Fluor, Chlor, einen geradkettigen oder verzweigten Alkoxy- oder Alkylthiorest mit jeweils 1 bis 2 Kohlenstoffatomen, einen Trifluormethoxy- oder Trifluormethylthiorest, für einen Hydroximinomethyl- oder Alkoximinomethylrest mit 1 bis 2 Kohlenstoffatomen im Alkylteil, für einen 1-Hydroximinoethyl- oder 1-Alkoxyiminoethylrest mit 1 bis 2 Kohlenstoffatomen im Alkoxyteil, für

einen jeweils gegebenenfalls einfach bis dreifach, gleich oder verschieden substituierten Phenyl-, Phenoxy-, Phenylthio-, Phenylmethoxy- oder Phenylmethylthiorest, wobei jeweils als Phenylsubstituenten genannt seien: Fluor, Chlor, Brom, Methyl, Hydroxy, Methoxy, Methylthio, Trifluormethyl, Trifluormethoxy, Trifluormethylthio und Hydroxycarbonyl,

Het für einen jeweils gegebenenfalls einfach bis dreifach, gleich oder verschieden substituierten Dioxolanyl- oder Dioxanylrest steht, wobei als Substituenten Methyl, Ethyl, n-Propyl, i-Propyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl in Frage kommen;

n eine der Zahlen 0, 1 oder 2 bedeutet,

m für 0 oder 1 steht; und

$R^2$ für Wasserstoff, einen geradkettigen oder verzweigten Alkylrest mit 1 bis 2 Kohlenstoffatomen, Allyl, Propargyl, Methylcarbonyl, Methoxycarbonyl, ferner für einen jeweils gegebenenfalls einfach bis dreifach, gleich oder verschieden substituierten Phenyl- oder Benzylrest, wobei als Substituenten die bei Y bereits genannten Phenylsubstituenten infrage kommen.

4. Verfahren zur Herstellung von Verbindungen gemäß Ansprüchen 1 bis 3, dadurch gekennzeichnet, daß man entweder

a) Imidazolyl-Verbindungen der Formel (II)

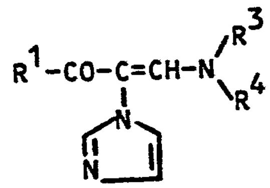

in welcher

$R^1$ die in Ansprüchen 1 bis 3 angegebene Bedeutung hat und

$R^3$ und $R^4$ gleich oder verschieden sind und für Alkylreste mit 1 bis 4 Kohlenstoffatomen stehen; oder gemeinsam mit dem N-Atom, an das sie gebunden sind, einen jeweils gegebenenfalls einfach bis dreifach durch Alkylreste mit 1 bis 4 Kohlenstoffatomen substituierten Piperidinyl-, Pyrrolidinyl- oder Morpholinylrest bilden,

mit Hydrazinen der Formel (III),

$$R^2\text{-NH-NH}_2$$

in welcher

$R^2$ die in Ansprüchen 1 bis 3 angegebene Bedeutung hat,

gegebenenfalls in Gegenwart eines Verdünnungsmittels umsetzt, oder

b) gegebenenfalls die nach dem Verfahren (a) erhältlichen Imidazolyl-pyrazol-Derivate der Formel (Ib),

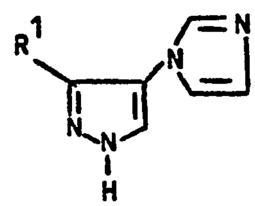

in welcher

$R^1$ die in Ansprüchen 1 bis 3 angegebene Bedeutung hat,

mit Halogeniden der Formel (IV)

$$Hal-R^5$$

in welcher

$R^5$ für die Bedeutungen von $R^2$ außer für Wasserstoff steht,

und Hal ein Halogenatom bedeutet,

in Gegenwart einer Base und gegebenenfalls in Gegenwart eines Verdünnungsmittels umsetzt

und an die so erhaltenen Verbindungen der Formel (I) gegebenenfalls anschließend eine Säure addiert.

5. Verbindungen nach Ansprüchen 1 bis 3 und deren Säureadditionssalze zur Verwendung bei der Bekämpfung von Kreislauferkrankungen.

6. Verbindungen nach Ansprüchen 1 bis 3 und deren Säureadditionssalze zur Verwendung bei der Therapie und Prophylaxe von thromboembolischen und ischämischen Erkrankungen.

7. Verwendung von Verbindungen nach Ansprüchen 1 bis 3 bzw. von deren Säureadditionssalzen bei der Bekämpfung von Kreislauferkrankungen.

8. Verwendung von Verbindungen nach Ansprüchen 1 bis 3 bzw. von deren Säureadditionssalzen bei der Therapie und Prophylaxe von thromboembolischen und ischämischen Erkrankungen.

9. Arzneimittel, gekennzeichnet durch einen Gehalt an Verbindungen nach Ansprüchen 1 bis 3 bzw. deren Säureadditionssalzen.

10. Verfahren zur Herstellung eines Arzneimittels nach Anspruch 9, dadurch gekennzeichnet, daß man eine Verbindung nach Ansprüchen 1 bis 3 bzw. deren Säureadditionssalz unter Verwendung üblicher Hilfs- und/oder Trägerstoffe in eine geeignete galenische Applikationsform überführt.